# EUROPEAN PATENT SPECIFICATION

(11) **EP 4 091 535 B1**
(45) Date of publication and mention of the grant of the patent: **15.05.2024**
(21) Application number: 22174223.2
(22) Date of filing: 19.05.2022
(51) Int. Cl.: A61B 3/13, A61B 3/032, A61B 3/09, A61B 3/103, A61B 3/15, A61B 3/00

(54) **SUBJECTIVE OPTOMETRY DEVICE**
SUBJEKTIVE OPTOMETRIEVORRICHTUNG
DISPOSITIF D'OPTOMÉTRIE SUBJECTIVE

(30) Priority: 21.05.2021 JP 2021086210
(43) Date of publication of application: 23.11.2022
(73) Proprietor: Nidek Co., Ltd., Gamagori-shi, Aichi, 443-0038 (JP)
(72) Inventor: IIDA, Yasumasa, Gamagori, Aichi (JP); KAMIKAWA, Tomohiro, Gamagori, Aichi (JP)
(74) Representative: Hoefer & Partner Patentanwälte mbB

(56) References cited:
- JP-A- 2021 019 956
- US-A1- 2018 256 022
- US-A1- 2018 296 085
- US-A1- 2019 150 735

## Description

### TECHNICAL FIELD

The present disclosure relates to a subjective optometry device for subjectively measuring an optical characteristic of a subject eye.

### BACKGROUND ART

A subjective optometry device for subjectively measuring optical characteristics of a subject eye is known. For example, in JP 2017- 086 652 A, a target light flux from a measurement unit is guided to the subject eye through a fixed optical member (concave mirror) that is fixedly arranged.

In the above configuration, since the measurement unit moves with respect to the fixedly arranged fixed optical member, there is a possibility that the optical characteristics of the subject eye cannot be measured accurately. For example, when the measurement unit moves significantly, a relationship between a distance from the subject eye to the fixed optical member and a distance from the measurement unit to the fixed optical member changes, and an optical magnification is likely to change. Further, for example, when the measurement unit moves significantly, optical aberrations are likely to occur.
Prior art document US 2018 / 256 022 A1 refers to a subjective optometry apparatus that includes a light projecting optical system that projects a target light flux to an examinee's eye, a fixed optical element that guides an image of the target light flux to the examinee's eye so as to have an optically predetermined examination length, a calibration optical system disposed in an optical path of the light projecting optical system to change optical characteristics of the examinee's eye, a measurement unit that accommodates the light projecting optical system; a positional information acquiring portion that acquires positional information of the measurement unit, a correction amount setting portion that sets a correction amount for correcting a projection magnification of the target light flux projected to the examinee's eye, based on the positional information, and a correction portion that corrects the projection magnification of the target light flux based on the correction amount set by the correction amount setting portion.
Prior art document US 2018 / 296 085 A1 discloses a subjective optometry apparatus includes a light projecting optical system which projects a target light flux toward an examinee's eye to project a visual target onto the examinee's eye, a calibration optical system which is disposed in an optical path from the light projecting optical system to the examinee's eye and changes optical characteristics of the target light flux, an optical member which guides the target light flux of which the optical characteristics is changed by the calibration optical system to the examinee's eye, a light deflection section being different from the calibration optical system, which includes light deflection members provided in a left and right pair and a driving section which rotationally drives the light deflection members, and deflects the target light flux by rotating the light deflection members, and a control section which controls the light deflection section based on prism information to deflect the target light flux.

### SUMMARY OF INVENTION

A technical object of the present disclosure is to provide a subjective optometry device capable of accurately measuring an optical characteristic of a subject eye.

The object underlying the present invention is achieved by a subjective optometry device according to independent claim 1. Preferred embodiments are defined in the respective dependent claims.

In order to solve the above-described problems, the present disclosure has the following configuration.
(1) A subjective optometry device for subjectively measuring an optical characteristic of a subject eye, the subjective optometry device including:
   a projection optical system that projects a target light flux toward the subject eye;
   a calibration optical system that changes an optical characteristic of the target light flux;
   a fixed optical member that is fixedly arranged in an optical path of the projection optical system and optically presents an image of the target light flux to the subject eye at a predetermined examination distance;
   a first moving means for moving a measurement unit with respect to the fixed optical member to perform first alignment of an optical axis of the projection optical system with respect to the subject eye, the measurement unit including at least the projection optical system;
   a second moving means for moving the measurement unit and the fixed optical member with respect to the subject eye, in a state of maintaining a positional relationship between the measurement unit and the fixed optical member, to perform second alignment such that the image of the target light flux is irradiated onto the subject eye at a predetermined optical magnification; and
   a movement control means for controlling the first moving means and the second moving means to perform alignment of the measurement unit with respect to the subject eye.
(2) In the subjective optometry device according to above-described (1),
   in which the movement control means controls the first moving means to perform the first alignment after controlling the second moving means to perform the second alignment.
(3) In the subjective optometry device according to above-described (1) or (2),
   in which the movement control means controls the second moving means to perform rough alignment of the measurement unit with respect to the subject eye and controls the first moving means to perform fine alignment of the measurement unit with respect to the subject eye, to adjust alignment between the subject eye and the measurement unit.
(4) In the subjective optometry device according to above-described (3),
   in which the movement control means controls the first moving means to perform tracking of the subject eye after performing the alignment between the subject eye and the measurement unit.
(5) In the subjective optometry device according to any one of above-described (1) to (4),
   in which the movement control means controls the second moving means to move the measurement unit and the fixed optical member at least in a front-rear direction with respect to the subject eye, and controls the first moving means to move the measurement unit at least in a left-right direction and an up-down direction with respect to the fixed optical member.
(6) In the subjective optometry device according to any one of above-described (1) to (5),
   in which the measurement unit has a left eye measurement unit and a right eye measurement unit provided as a left and right pair,
   the fixed optical member is shared in an optical path of a target light flux from the left eye measurement unit and in an optical path of a target light flux from the right eye measurement unit, and
   the second moving means integrally moves the left eye measurement unit, the right eye measurement unit, and the fixed optical member.
(7) In the subjective optometry device according to any one of above-described (1) to (6), further including:
   a position information acquisition means for acquiring position information of the measurement unit; and
   a correction means for correcting an optical aberration, which is generated on an account that the target light flux passes through the fixed optical member, based on the position information.
(8) In the subjective optometry device according to any one of above-described (1) to (7),
   in which the measurement unit includes the calibration optical system with the projection optical system, and
   the fixed optical member guides the target light flux calibrated by the calibration optical system to the subject eye.
(9) In the subjective optometry device according to any one of above-described (1) to (8),
   in which the fixed optical member is a concave mirror.

### BRIEF DESCRIPTION OF DRAWINGS

FIG. 1 is an external view of a subjective optometry device.
FIG. 2 is a view showing a left eye measurement unit.
FIG. 3 is a schematic view when the inside of the device is viewed from the front.
FIG. 4 is a schematic view when the inside of the device is viewed from the side.
FIG. 5 is a schematic view when the inside of the device is viewed from above.
FIG. 6 is a simplified view in which optical members inside the device are arranged on a straight line.
FIG. 7 is a view showing a control system.
FIG. 8 is a view showing positions of a first unit and a second unit with respect to a subject eye.
FIG. 9 is a view showing a position of the first unit with respect to a concave mirror.

### DESCRIPTION OF EMBODIMENTS

### <Overview>

The overview of a subjective optometry device according to an embodiment of the present disclosure will be described. In the present embodiment, a left-right direction in the subjective optometry device is an X direction, an up-down direction in the subjective optometry device is a Y direction, and a front-rear direction in the subjective optometry device (working distance direction) is a Z direction. L and R attached to the reference numerals indicate those for the left eye and those for the right eye, respectively. In addition, the items classified by <> below can be used independently or in association with each other.

The subjective optometry device of the present embodiment subjectively measures an optical characteristic of a subject eye. For example, at least one of the eye refractive power (spherical power, cylindrical power, astigmatism axis angle, and the like), contrast sensitivity, binocular vision function (amount of phoria and stereoscopic vision function) and the like may be measured. The subjective optometry device includes a measuring means (for example, measurement unit 7). For example, the measuring means may include a subjective measuring means. In addition, for example, the measuring means may include an objective measuring means. In the present embodiment, the measuring means may include at least a subjective measuring means, and may include both a subjective measuring means and an objective measuring means.

### <Subjective measuring means>

The subjective optometry device may include a subjective measuring means (for example, subjective measurement optical system 25). For example, the subjective measuring means has at least a projection optical system (for example, projection optical system 30) that projects a target light flux toward the subject eye. Furthermore, the subjective measuring means may include a calibration optical system (for example, calibration optical system 60) that changes an optical characteristic of a target light flux.

### <Projection optical system>

The projection optical system projects the target light flux toward the subject eye. The projection optical system may have at least one optical member that guides the target light flux toward the subject eye.

The projection optical system may include a target presenting means. The target presenting means presents the target to the subject eye. In this case, the projection optical system projects the target light flux emitted from the target presenting means toward the subject eye. For example, a display (for example, display 31) can be used as the target presenting means. Further, for example, a light source and a digital micromirror device (DMD) can be used as the target presenting means. In general, the DMD has high reflectance and is bright, and thus, the quantity of the target light flux can be maintained as compared with the case of using an LCD. Further, for example, as the target presenting means, a visible light source for presenting the target and a target plate can be used. The target plate is a rotatable disc plate and may have a plurality of targets. The targets are switched and arranged by rotating the target plate by a motor or the like on the optical path where the target light flux is guided to the subject eye.

The projection optical system may include a left eye projection optical system and a right eye projection optical system which are provided as a left and right pair. For example, members that form the left eye projection optical system and members that form the right eye projection optical system may be the same member, or at least some of the members may be different members. Further, for example, at least some of the members that form the left eye projection optical system and the members that form the right eye projection optical system may be shared.

### <Calibration optical system>

The calibration optical system is arranged in the optical path of the projection optical system, and changes the optical characteristic (at least one of spherical power, cylindrical power, astigmatism axis angle, polarization characteristics, aberration amount, and the like) of the target light flux.

For example, the calibration optical system may be able to change at least one of the spherical power, the cylindrical power, the astigmatism axis angle, and the like of the target light flux by controlling an optical element. The optical element may be at least one of a spherical lens, a cylindrical lens, a cross cylinder lens, a rotary prism, a wavefront modulation element, a varifocal lens, and the like. It is needless to say that an optical element different from these optical elements may be used.

Further, for example, the calibration optical system may calibrate the spherical power of the subject eye by optically changing a presentation distance of the target with respect to the subject eye. In this case, in order to optically change the presentation distance of the target, the target presenting means may be moved in the optical axis direction. In this case, in order to optically change the presentation distance of the target, an optical element (for example, spherical lens) arranged in the optical path may be moved in the optical axis direction.

The calibration optical system may be a combination of a configuration for controlling the optical element, a configuration for moving the target presenting means in the optical axis direction, and a configuration for moving the optical element arranged in the optical path in the optical axis direction.

In the present embodiment, the calibration optical system may be an eye refractive power measurement unit (phoropter) in which an optical element is arranged in front of the subject eye. For example, the eye refractive power measurement unit may have a varifocal lens and may change the eye refractive power of the varifocal lens. Further, for example, the eye refractive power measurement unit may include a lens disk in which a plurality of optical elements are arranged on the same circumference, and a driving means (for example, motor) for rotating the lens disk, and may electrically switch the optical elements by the drive of the driving means. It is needless to say that the eye refractive power measurement unit may include a varifocal lens, a lens disk, and a driving means. With these configurations, the target light flux directed toward the subject eye is projected through the eye refractive power measurement unit.

Further, in the present embodiment, the calibration optical system may change the optical characteristic of the target light flux by arranging the optical elements between the target presenting means and the optical member for guiding the target light flux from the projection optical system toward the subject eye, and by controlling the optical elements. In other words, the calibration optical system may be configured as a phantom lens refractometer (phantom calibration optical system). In this case, the target light flux calibrated by the calibration optical system is guided to the subject eye through the optical member.

The calibration optical system may include a left eye calibration optical system and a right eye calibration optical system which are provided as a left and right pair. For example, members that form the left eye calibration optical system and members that form the right eye calibration optical system may be the same member, or at least some of the members may be different members. Further, for example, at least some members that form the left eye calibration optical system and members that form the right eye calibration optical system may be shared.

### <Objective measuring means>

The subjective optometry device may include an objective measuring means (for example, objective measurement optical system 10) for objectively measuring the optical characteristic of the subject eye. For example, the objective measuring means may include a light projecting optical system (for example, light projecting optical system 10a) that projects a measured light flux to fundus of the subject eye, and a light receiving optical system (for example, light receiving optical system 10b) that receives a reflected light flux which is the measured light flux reflected at the fundus. In addition, as the objective optical characteristic, at least one of the eye refractive power (for example, spherical power, cylindrical power, astigmatism axis angle, and the like), the eye axial length, the corneal shape, and the like may be measured.

### <Fixed optical member>

The subjective optometry device includes a fixed optical member. The fixed optical member is a fixed optical member that is fixedly arranged in the optical path of the projection optical system, and is a fixed optical member that optically presents the image of the target light flux to the subject eye at a predetermined examination distance. For example, the fixed optical member may guide the target light flux from the projection optical system to the subject eye. Further, for example, the fixed optical member may guide the target light flux which is projected from the projection optical system and further calibrated by the calibration optical system to the subject eye. The fixed optical member may be shared in the optical path of the target light flux from a left eye measurement unit (described later) and in the optical path of the target light flux from a right eye measurement unit (described later). For example, as the fixed optical member, at least one of a concave mirror (for example, concave mirror 85), a lens, and the like can be used.

In the present embodiment, a concave mirror is used to optically present the image of the target light flux to the subject eye at a predetermined examination distance. Accordingly, it is not necessary to arrange a predetermined optical member at an actual distance, and thus, the space occupied by the device can be reduced.

### <First moving means>

The subjective optometry device includes a first moving means (for example, first moving unit 9). The first moving means aligns an optical axis of the projection optical system with the subject eye by moving the measurement unit (for example, first unit A1) with respect to the fixed optical member. In the present embodiment, the first moving means may be capable of moving at least the measurement unit in the X direction and the Y direction. For example, the subject eye is likely to move in the XY direction, and there is a possibility that the target cannot be appropriately presented due to a displacement of the optical axis from the subject eye, but such a configuration reduces the possibility. A movement of the subject eye in the Z direction can also be dealt with by using a second moving means described later, but it is needless to say that the first moving means may be capable of moving the measurement unit in the Z direction. In other words, the first moving means may move the measurement unit in the three-dimensional direction with respect to the fixed optical member. Accordingly, the optical axis of the projection optical system can be arranged at a more appropriate position according to the movement of the subject eye.

For example, the measurement unit may be a unit including at least the projection optical system. As an example, the measurement unit may be configured as a measurement unit including the projection optical system, or may be configured as a measurement unit including the projection optical system and the calibration optical system. It is needless to say that the measurement unit may be configured as a measurement unit including other optical members together with the projection optical system. The measurement unit may include a left eye measurement unit and a right eye measurement unit provided as a left and right pair. The left eye measurement unit includes a left eye projection optical system (and left eye calibration optical system) and the right eye measurement unit includes a right eye projection optical system (and right eye calibration optical system).

In the present embodiment, the first moving means may be used to detect a line of sight of the subject eye and align the line of sight (that is, visual axis) with the optical axis of the projection optical system. The line of sight of the subject eye may be at the position of the line of sight in which the subject eye gazes at the image of the target light flux. For example, a position of the line of sight based on a pupillary distance of the subject eye, a position of the line of sight based on a convergence angle according to the optical presentation distance of the target to the subject eye, and the like may be used. It is needless to say that a position of the line of sight based on both the pupillary distance and the convergence angle of the subject eye may be used.

### <Second moving means>

The subjective optometry device includes the second moving means (for example, second moving unit 8). The second moving means moves the measurement unit and the fixed optical member with respect to the subject eye, in a state of maintaining the positional relationship between the measurement unit and the fixed optical member, to perform alignment such that the image of the target light flux is irradiated onto the subject eye at a predetermined optical magnification. In other words, by integrally moving the measurement unit and the fixed optical member with respect to the subject eye, the image of the target light flux is aligned to be irradiated onto the subject eye at a predetermined optical magnification. Accordingly, an optical magnification of the image of the target light flux irradiated onto the subject eye is maintained. In the present embodiment, the second moving means may be capable of integrally moving at least the measurement unit and the fixed optical member in the Z direction. For example, when the subject eye moves in the Z direction, the optical magnification is likely to change, and there is a possibility that the target cannot be appropriately presented, but such a configuration reduces the possibility.

It is needless to say that the second moving means may be capable of moving the measurement unit and the fixed optical member in the X direction and the Y direction. In other words, the second moving means may integrally move the measurement unit and the fixed optical member in the three-dimensional direction with respect to the subject eye. Further, in the present embodiment, the second moving means may integrally move the left and right eye measurement units and the fixed optical member with respect to the subject eye.

### <Movement control means>

The subjective optometry device includes a movement control means (for example, control unit 70). The movement control means controls the first moving means and the second moving means to perform alignment of the measurement unit with respect to the subject eye. For example, the movement control means may perform the control of the first moving means and the control of the second moving means at the same timing. In addition, for example, the movement control means may perform the control of the first moving means and the control of the second moving means at the same timing. In this case, any of the control of the first moving means and the control of the second moving means may be executed first. Accordingly, the subject eye and the optical axis of the projection optical system match each other, and the image of the target light flux can be irradiated to the subject eye at a predetermined optical magnification.

In the present embodiment, the movement control means may control the second moving means to perform the alignment and then control the first moving means to perform the alignment. In this case, first, by integrally moving the measurement unit and the fixed optical member with respect to the subject eye in a state where the positional relationship between the measurement unit and the fixed optical member is maintained, and by adjusting the optical magnification, an image of an appropriate target light flux can be irradiated onto the subject eye. Subsequently, by moving the measurement unit with respect to the subject eye (with respect to the fixed optical member), and by making the optical axis of the projection optical system match with the subject eye, an appropriate image of the target light flux can be irradiated onto the subject eye. By performing the alignment by controlling the second moving means first, the movement of the measurement unit by controlling the first moving means can be minimized, and thus the optical aberrations can be further reduced.

In addition, in the present embodiment, the movement control means may control the second moving means to perform rough alignment of the measurement unit with respect to the subject eye and control the first moving means to perform fine alignment of the measurement unit with respect to the subject eye, to adjust alignment between the subject eye and the measurement unit. In other words, the rough alignment of the measurement unit with respect to the subject eye may be performed by controlling the second moving means, and the fine alignment of the measurement unit with respect to the subject eye may be performed by controlling the first moving means. For example, by making the optical magnifications match each other by the rough alignment and then making the subject eye match with the optical axis of the projection optical system by the fine alignment, the occurrence of optical aberrations is minimized, and an appropriate target can be presented to the subject eye.

The movement control means may further control the first moving means to perform tracking of the subject eye after performing alignment (rough alignment and fine alignment) between the subject eye and the measurement unit. For example, the measurement unit may be moved according to the movement of the subject eye (for example, fixational eye movement, and the like) during the subjective measurement. By quickly responding to the movement of the subject eye and making the subject eye and the optical axis of the projection optical system appropriately match each other, an appropriate target can be presented to the subject eye.

Further, in the present embodiment, the movement control means may control the second moving means to move the measurement unit and the fixed optical member with respect to the subject eye at least in the Z direction, and may control the first moving means to move the measurement unit with respect to the fixed optical member at least in the X direction and Y direction. Accordingly, a predetermined optical magnification can be maintained according to the displacement of the subject eye in the Z direction. In addition, the target can be appropriately presented to the subject eye according to the displacement of the subject eye in the XY direction (that is, the displacement of the optical axis with respect to the subject eye).

### <Position information acquisition means>

The subjective optometry device includes a position information acquisition means (for example, control unit 70). The position information acquisition means acquires position information of the measurement unit. For example, the position information of the measurement unit may be expressed as position coordinates of the measurement unit. Further, for example, the position information of the measurement unit may be expressed as a three-dimensional distance from the subject eye to the measurement unit, a three-dimensional distance from the fixed optical member to the measurement unit, and the like.

In the present embodiment, the measurement unit integrally moves as a moving unit (for example, second unit A2) together with the fixed optical member. For example, the measurement unit is provided inside the moving unit and moves in the moving unit by controlling the first moving means. Therefore, the position information acquisition means may acquire the position information of the measurement unit with respect to the position information of the moving unit. More specifically, the position coordinates of the measurement unit with respect to the moving unit may be obtained, or the three-dimensional distance with respect to the moving unit may be obtained.

### <Correction means>

The subjective optometry device includes a correction means (for example, control unit 70). The correction means corrects the optical aberrations generated by the target light flux passing through the fixed optical member, based on the position information of the measurement unit. For example, the correction means may correct the optical aberrations by performing arithmetic processing based on the optical characteristic of the subject eye and the position information of the measurement unit. Further, for example, the correction means may correct the optical aberrations by referring to a correction table based on the optical characteristic of the subject eye and the position information of the measurement unit. The correction table may be stored in advance in storage means (for example, memory 75). Accordingly, for example, even when the relationship between the optical magnifications is broken and optical aberrations occur, the subject eye and the optical axis of the projection optical system are appropriately aligned.

For example, the correction means may include a correction amount setting means. The correction amount setting means may set a correction amount for correcting the optical aberrations generated by the target light flux passing through the fixed optical member. In this case, as an example, the correction means may correct the optical aberrations by controlling at least one of the projection optical system and the calibration optical system and by changing the optical characteristic of the target light flux based on the correction amount. In addition, such a correction amount may be obtained by arithmetic processing or a correction table based on the optical characteristic of the subject eye and the position information of the measurement unit. Accordingly, it is possible to accurately acquire the optical characteristic of the subject eye.

### <Example>

An example of a subjective optometry device (hereinafter, referred to as optometry device) according to the present embodiment will be described.

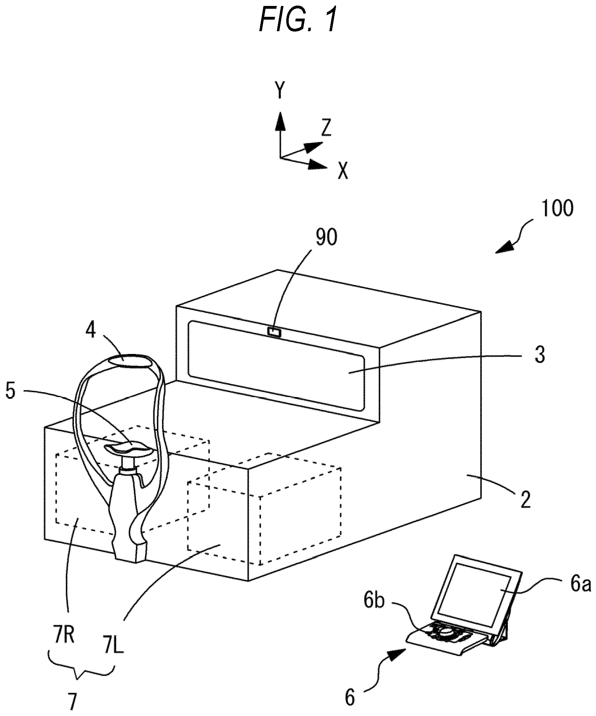

FIG. 1 is an external view of an optometry device 100. For example, the optometry device 100 includes a housing 2, a presentation window 3, a forehead pad 4, a chin support 5, a controller 6, an imaging unit 90, and the like. The housing 2 has a measurement unit 7, a deflection mirror 81, a reflection mirror 84, a concave mirror 85, and the like therein. The presentation window 3 is used to present a target to a subject eye E. The forehead pad 4 and the chin support 5 are used to keep a distance between the subject eye E and the optometry device 100 constant. The controller 6 includes a monitor 6a, a switch unit 6b, and the like. The monitor 6a displays various information (for example, the measurement result of the subject eye). The monitor 6a may be a touch panel that also has the function of the switch unit 6b. The switch unit 6b is used to make various settings (for example, input of a start signal). The signal corresponding to an operation instruction from the controller 6 is output to a control unit 70 by wired communication or wireless communication.

The imaging unit 90 is used to capture a face of an examinee and adjust the position of the subject eye in the Y direction. The imaging unit 90 includes an imaging optical system (not shown). For example, the imaging optical system may include an imaging element and a lens. In the present example, the imaging unit 90 is provided in a second unit A2, which will be described later, and moves in the Z direction together with the second unit A2.

### <Measurement unit>

The measurement unit 7 includes a left eye measurement unit 7L and a right eye measurement unit 7R. The left eye measurement unit 7L and the right eye measurement unit 7R are configured with the same members. It is needless to say that at least some parts of the left eye measurement unit 7L and the right eye measurement unit 7R may be configured with different members. The measurement unit 7 includes a pair of left and right subjective measurement units, and a pair of left and right objective measurement units (described later in detail). A target light flux and a measured light flux from the measurement unit 7 are guided to the subject eye E through the presentation window 3.

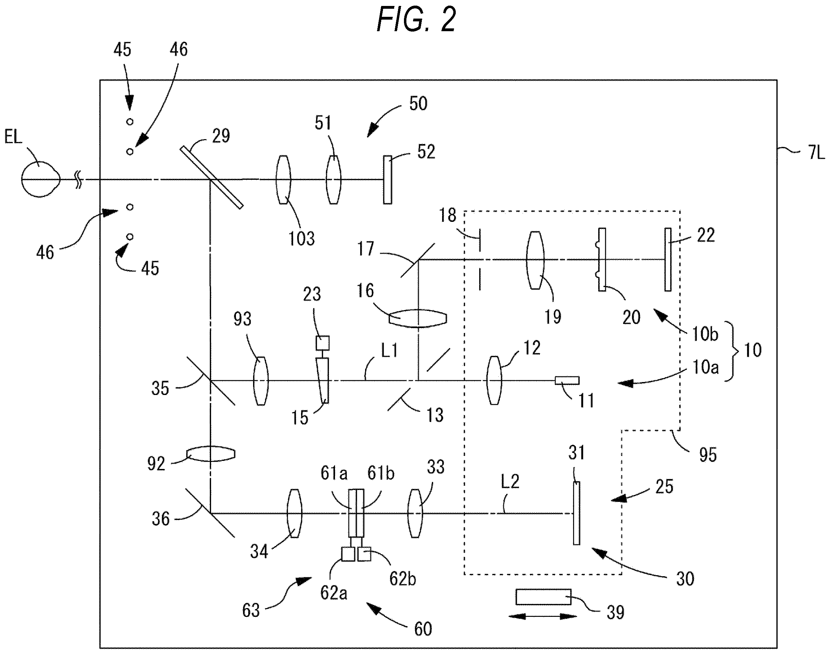

FIG. 2 is a view showing the left eye measurement unit 7L. Since the right eye measurement unit 7R has the same configuration as that of the left eye measurement unit 7L, the description thereof will be omitted. For example, the left eye measurement unit 7L includes an objective measurement optical system 10, a subjective measurement optical system 25, a first index light projecting optical system 45, a second index light projecting optical system 46, an observation optical system 50, and the like.

### <Objective measurement optical system>

The objective measurement optical system 10 is used as a part of the configuration of the objective measurement unit that objectively measures the optical characteristic of the subject eye. In the present example, the eye refractive power of the subject eye E is measured as optical characteristic of the subject eye E. For example, the objective measurement optical system 10 includes the light projecting optical system 10a and the light receiving optical system 10b.

The light projecting optical system 10a projects a spot-shaped target onto the fundus of the subject eye E through the pupil center part of the subject eye E. For example, the light projecting optical system 10a includes a light source 11, a relay lens 12, a hole mirror 13, a prism 15, an objective lens 93, a dichroic mirror 35, a dichroic mirror 29, and the like. The light source 11 emits a measured light flux. The light source 11 has a conjugate relationship with the fundus of the subject eye E. A hole portion of the hole mirror 13 has a conjugate relationship with the pupil of the subject eye E. The prism 15 is a light flux deflection member. The prism 15 is arranged at a position displaced from a position conjugate with the pupil of the subject eye E, and the measured light flux passing through the prism 15 is eccentric with respect to an optical axis L1. The prism 15 is rotationally driven by a driving unit (motor) 23 around the optical axis L1. The dichroic mirror 35 makes the optical path of the objective measurement optical system 10 and the optical path of the subjective measurement optical system 25 a common optical path. In other words, the optical axis L1 of the objective measurement optical system 10 and the optical axis L2 of the subjective measurement optical system 25 are coaxial with each other. The dichroic mirror 29 is an optical path branching member. The dichroic mirror 29 reflects the measured light flux by the light projecting optical system 10a and the target light flux by the projection optical system 30 (described later), and guides the reflected measured light flux and target light flux to the subject eye E.

The light receiving optical system 10b takes out the fundus reflected light flux reflected by the fundus of the subject eye E in a ring shape through the peripheral portion of the pupil of the subject eye E. For example, the light receiving optical system 10b includes the dichroic mirror 29, the dichroic mirror 35, the objective lens 93, the prism 15, the hole mirror 13, a relay lens 16, a mirror 17, a light receiving diaphragm 18, a collimator lens 19, a ring lens 20, an imaging element 22, and the like. The ring lens 20 includes a ring-shaped lens unit and a light-shielding unit in which a light-shielding coating is applied to a region other than the lens unit. The ring lens 20 has a positional relationship optically conjugate with the pupil of the subject eye E. The light receiving diaphragm 18 and the imaging element 22 have a conjugate relationship with the fundus of the subject eye E. The output from the imaging element 22 is input to the control unit 70.

Further, in the present example, the prism 15 is arranged on the common optical axis of the light projecting optical system 10a and the light receiving optical system 10b. For example, the measured light flux from the light projecting optical system 10a passes through the prism 15 and is incident on the subject eye E, and the fundus reflected light flux reflected by the fundus of the subject eye E passes through the same prism 15, and thus, in the optical system after this, a reverse scanning is performed as if there was no eccentricity of the irradiated light flux and the fundus reflected light flux (light receiving light flux) on the pupil.

### <Subjective measurement optical system>

The subjective measurement optical system 25 is used as a part of the configuration of the subjective measurement unit that subjectively measures the optical characteristic of the subject eye E. In the present example, the eye refractive power of the subject eye E is measured as optical characteristic of the subject eye E. For example, the subjective measurement optical system 25 includes the projection optical system 30 and the calibration optical system 60.

### <Projection optical system>

The projection optical system 30 projects a target light flux toward the subject eye E. For example, the projection optical system 30 includes the display 31, a projective lens 33, a projective lens 34, a reflection mirror 36, an objective lens 92, the dichroic mirror 35, the dichroic mirror 29, and the like. A target (fixation target, examination target, and the like) is displayed on the display 31.

### <Calibration optical system>

The calibration optical system 60 is arranged in the optical path of the projection optical system 30. Further, the calibration optical system 60 changes the optical characteristic of the target light flux emitted from the display 31. For example, the calibration optical system 60 includes an astigmatism calibration optical system 63, a drive mechanism 39, and the like. The astigmatism calibration optical system 63 is used to calibrate the cylindrical power and the astigmatism axis angle of the subject eye E. The astigmatism calibration optical system 63 is arranged between the projective lens 33 and the projective lens 34. The astigmatism calibration optical system 63 includes two positive cylindrical lens 61a and 61b, having the same focal length. Each of the cylindrical lens 61a and the cylindrical lens 61b rotates independently around the optical axis L2 by driving a rotation mechanism 62a and a rotation mechanism 62b.

Further, in the present example, a configuration in which the cylindrical lens 61a and the cylindrical lens 61b are used as the astigmatism calibration optical system 63 has been described as an example, but the present invention is not limited thereto. The astigmatism calibration optical system 63 may have a configuration capable of calibrating the cylindrical power, the astigmatism axis angle, and the like. As an example, a calibration lens may be taken in and out of the optical path of the projection optical system 30.

The light source 11 and the relay lens 12 included in the light projecting optical system 10a, the light receiving diaphragm 18 included in the light receiving optical system 10b, the collimator lens 19, a ring lens 20, the imaging element 22, and the display 31 included in the projection optical system 30 can be integrally moved in the optical axis direction by the drive mechanism 39. In other words, the display 31, the light source 11, the relay lens 12, the light receiving diaphragm 18, the collimator lens 19, the ring lens 20, and the imaging element 22 are synchronized as a driving unit 95 and are integrally moved by the drive mechanism 39. The drive mechanism 39 includes a motor and a slide mechanism.

The drive mechanism 39 moves the display 31 in the optical axis L2 direction by moving the driving unit 95 in the optical axis direction. Accordingly, in the objective measurement, cloud fog can be applied to the subject eye E. In the subjective measurement, the presentation distance of the target to the subject eye E can be optically changed to calibrate the spherical power of the subject eye E. In other words, the configuration in which the display 31 is moved in the direction of the optical axis L2 is used as a spherical calibration optical system for calibrating the spherical power of the subject eye E, and the spherical power of the subject eye E is calibrated by changing the position of the display 31. The configuration of the spherical calibration optical system may be different from that of the present example. For example, the spherical power may be calibrated by arranging multiple optical elements in the optical path. Further, for example, the spherical power may be calibrated by arranging the lens in the optical path and moving the lens in the optical axis direction.

Further, the drive mechanism 39 moves the light source 11, the relay lens 12, and the members from the light receiving diaphragm 18 to the imaging element 22 in the optical axis L1 direction by moving the driving unit 95 in the optical axis direction. Accordingly, the light source 11, the light receiving diaphragm 18, and the imaging element 22 are arranged to be optically conjugated with respect to the fundus of the subject eye E. Regardless of the movement of the driving unit 95, the hole mirror 13 and the ring lens 20 are arranged to be conjugated with the pupil of the subject eye E at a constant magnification. Therefore, the fundus reflected light flux which is a reflection of the measured light flux of the light projecting optical system 10a is always incident on the ring lens 20 of the light receiving optical system 10b as a parallel light flux, and a ring-shaped light flux having the same size as the ring lens 20 is imaged on the imaging element 22 in a focused state, regardless of the eye refractive power of the subject eye E.

### <First index light projecting optical system and second index light projecting optical system>

The first index light projecting optical system 45 and the second index light projecting optical system 46 are arranged between the dichroic mirror 29 and the deflection mirror 81 (described later). The first index light projecting optical system 45 emits near infrared light for projecting a finite-distance alignment index onto the cornea of the subject eye E. The second index light projecting optical system 46 is arranged at a position different from that of the first index light projecting optical system 45, and emits near infrared light for projecting a finite-distance alignment index onto the cornea of the subject eye. The near infrared light (alignment light) emitted from the second index light projecting optical system 46 is also used as the anterior eye part capturing light for capturing the anterior eye part of the subject eye by the observation optical system 50.

### <Observation optical system>

The observation optical system (imaging optical system) 50 includes the dichroic mirror 29, an objective lens 103, an imaging lens 51, an imaging element 52, and the like. The dichroic mirror 29 transmits the anterior eye part observation light and the alignment light. The imaging element 52 has an imaging surface arranged at a position conjugated with the anterior eye part of the subject eye E. The output from the imaging element 52 is input to the control unit 70. Accordingly, the anterior eye part image of the subject eye E is captured by the imaging element 52 and displayed on the monitor 6a. The observation optical system 50 also serves as an optical system for detecting the alignment index image formed on the cornea of the subject eye E by the first index light projecting optical system 45 and the second index light projecting optical system 46, and the position of the alignment index image is detected by the control unit 70.

### <Internal configuration of optometry device>

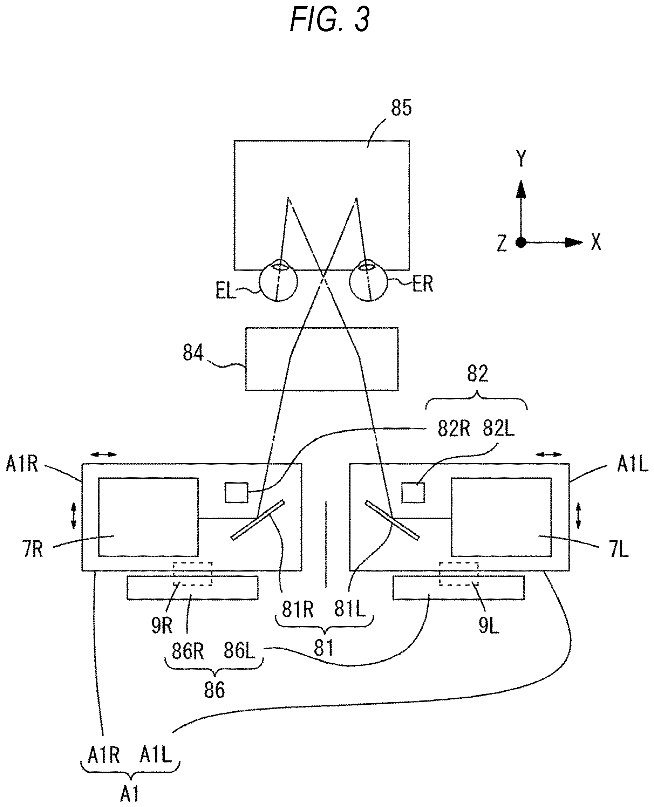

Next, an internal configuration of the optometry device 100 will be described. FIG. 3 is a schematic view of the inside of the optometry device 100 when viewed from the front.

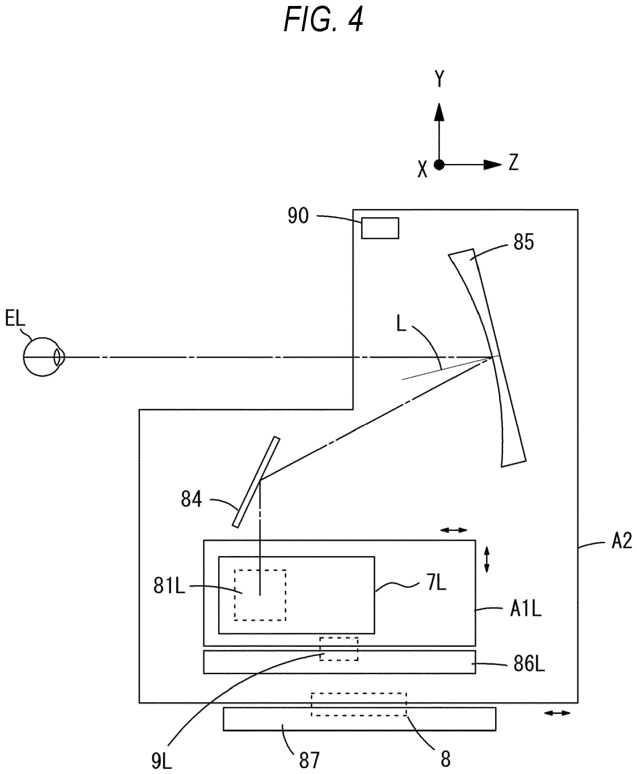

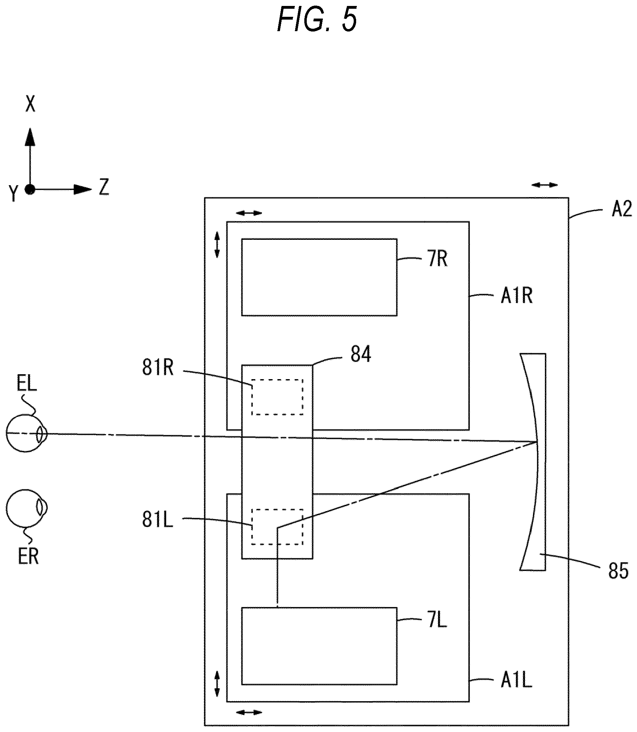

FIG. 4 is a schematic view of the inside of the optometry device 100 when viewed from the side. FIG. 5 is a schematic view of the inside of the optometry device 100 when viewed from above. In addition, FIGs. 4 and 5 show only the optical axis of the left eye measurement unit 7L for convenience of description.

The optometry device 100 includes the objective measurement unit. For example, the objective measurement unit includes the measurement unit 7, the deflection mirror 81, the reflection mirror 84, the concave mirror 85, and the like. In addition, the optometry device 100 includes the subjective measurement unit. For example, the subjective measurement unit includes the measurement unit 7, the deflection mirror 81, the reflection mirror 84, the concave mirror 85, and the like. The objective measurement unit and the subjective measurement unit is not limited to this configuration. For example, the configuration may not include the reflection mirror 84. In this case, the light flux from the measurement unit 7 may be emitted in the oblique direction with respect to an optical axis L of the concave mirror 85 after passing through the deflection mirror 81. Further, for example, a configuration including a half mirror may be included. In this case, the light flux from the measurement unit 7 may be emitted in the oblique direction with respect to the optical axis L of the concave mirror 85 through the half mirror.

For example, the deflection mirror 81 includes a left eye deflection mirror 81L and a right eye deflection mirror 81R, which are respectively provided as a left and right pair. For example, the deflection mirror 81 is arranged between the calibration optical system 60 and the subject eye E. In other words, the calibration optical system 60 in the present example has a left eye calibration optical system and a right eye calibration optical system provided as a left and right pair, the left eye deflection mirror 81L is arranged between the left eye calibration optical system and a left eye EL, and the right eye deflection mirror 81R is arranged between the right eye calibration optical system and a right eye ER. For example, the deflection mirror 81 is preferably arranged at the pupil conjugate position.

For example, the left eye deflection mirror 81L reflects the light flux irradiated from the left eye measurement unit 7L and guides the light flux to the left eye EL. Further, for example, the left eye deflection mirror 81L reflects the fundus reflected light flux from the left eye EL and guides the fundus reflected light flux to the left eye measurement unit 7L. For example, the right eye deflection mirror 81R reflects the light flux irradiated from the right eye measurement unit 7R and guides the light flux to the right eye ER. Further, for example, the right eye deflection mirror 81R reflects the fundus reflected light flux from the right eye ER and guides the fundus reflected light flux to the right eye measurement unit 7R.

For example, the deflection mirror 81 is rotationally moved by a driving unit 82. For example, by rotating and moving the deflection mirror 81, an apparent light flux for forming an image of the target light flux in front of the subject eye is deflected, and the formation position of the image of the target light flux can be optically corrected. For example, the driving unit 82 includes a motor or the like. For example, the driving unit 82 rotates the deflection mirror 81 with respect to the rotation axis in the horizontal direction (X direction) and the rotation axis in the vertical direction (Y direction). In other words, the driving unit 82 rotates the deflection mirror 81 in the XY directions. The rotation of the deflection mirror 81 may be in either the horizontal direction or the vertical direction. For example, the driving unit 82 includes a driving unit 82L for driving the left eye deflection mirror 81L and a driving unit 82R for driving the right eye deflection mirror 81R.

Further, in the present example, a configuration in which the deflection mirror 81 is used as a deflection member that reflects the light flux irradiated from the measurement unit 7 and guides the light flux to the subject eye E has been described as an example, but the present invention is not limited thereto. The deflection member may be a prism, a lens, or the like, as long as the deflection member can reflect the light flux irradiated from the measurement unit 7 and guide the light flux to the subject eye E.

For example, a plurality of deflection mirrors 81 may be provided in each of the left eye optical path and the right eye optical path. For example, a configuration in which two deflection mirrors are provided in each of the left eye optical path and the right eye optical path (for example, a configuration in which two deflection mirrors are provided in the left eye optical path) can be employed. In this case, one deflection mirror may be rotated in the X direction and the other deflection mirror may be rotated in the Y direction.

For example, the concave mirror 85 guides the target light flux that has passed through the calibration optical system 60 to the subject eye E, and forms an image of the target light flux that has passed through the calibration optical system 60 in front of the subject eye E. For example, the concave mirror 85 is shared in the left eye measurement unit 7L and in the right eye measurement unit 7R. For example, the concave mirror 85 is shared in the left eye optical path including the left eye calibration optical system and in the right eye optical path including the right eye calibration optical system. In other words, the concave mirror 85 is arranged at a position where both the left eye optical path including the left eye calibration optical system and the right eye optical path including the right eye calibration optical system pass through. It is needless to say that the concave mirror 85 may not be shared in the left eye optical path and in the right eye optical path. In other words, a concave mirror may be provided in each of the left eye optical path including the left eye calibration optical system and the right eye optical path including the right eye calibration optical system.

### <Optical path of obj ective measurement unit and subjective measurement unit>

The optical path of the objective measurement unit will be described by taking the left eye optical path as an example. The right eye optical path has the same configuration as that of the left eye optical path. The measured light flux emitted from the light source 11 of the light projecting optical system 10a reaches the left eye EL through each optical member. For example, the measured light flux is guided from the left eye measurement unit 7L to the left eye deflection mirror 81L, by sequentially passing through the optical members from the relay lens 12 to the dichroic mirror 29. Furthermore, the target light flux is reflected by the left eye deflection mirror 81L, and is guided to the left eye EL through the reflection mirror 84 and the concave mirror 85. A spot-shaped point light source image is formed on the fundus of the left eye EL. At this time, the prism 15 rotating around the optical axis causes the pupil projection image (irradiated light flux on the pupil) of the hole portion in the hole mirror 13 to be eccentrically rotated at high speed.

At the fundus of the subject eye E, the measured light flux is reflected and emitted, and is guided to the left eye measurement unit 7L through the concave mirror 85, the reflection mirror 84, and the deflection mirror 81. Furthermore, when the light is reflected by the dichroic mirror 29 and the dichroic mirror 35 and is collected by the objective lens 93, and the light is collected again on the opening of the light receiving diaphragm 18 through the prism 15 which rotates at high speed and the optical members from the hole mirror 13 to the mirror 17, the collimator lens 19 and the ring lens 20 form an image on the imaging element 22 as a ring-shaped image. By analyzing the ring-shaped image captured by the imaging element 22, the optical characteristic of the subject eye E can be objectively measured.

The optical path of the subjective measurement unit will be described by taking the left eye optical path as an example. The right eye optical path has the same configuration as that of the left eye optical path. The target light flux emitted from the display 31 of the subjective measurement optical system 25 reaches the left eye EL through each optical member. For example, the target light flux is guided from the left eye measurement unit 7L to the left eye deflection mirror 81L, by sequentially passing through the optical members from the projective lens 33 to the dichroic mirror 29. Furthermore, the target light flux is reflected by the left eye deflection mirror 81L, and is guided to the left eye EL through the reflection mirror 84 and the concave mirror 85.

Accordingly, an image of the target light flux calibrated by the calibration optical system 60 is formed on the fundus of the left eye EL with reference to the spectacles wearing position (for example, approximately 12 mm from the corneal apex position) of the left eye EL. Therefore, the adjustment of the spherical power by the calibration optical system (in the present example, the drive of the drive mechanism 39) in front of the eyes is equivalent to the arrangement of the astigmatism calibration optical system 63 as if in front of the eyes. The examinee can collimate the image of the target light flux formed in front of the eyes optically at a predetermined examination distance through the concave mirror 85, in a natural state.

### <First unit and second unit>

In the present example, the inside of the optometry device 100 includes a first unit A1 and a second unit A2. For example, the first unit includes a left eye unit A1L and right eye unit A1R which are provided as a left and right pair. The left eye unit A1L is a unit for integrally moving the left eye measurement unit 7L and the left eye deflection mirror 81L. In other words, the left eye unit A1L is a unit for integrally moving the projection optical system 30 and the calibration optical system 60 included in the left eye measurement unit 7L, and the left eye deflection mirror 81L. Similarly, the right eye unit A1R is a unit for integrally moving the right eye measurement unit 7R and the right eye deflection mirror 81R. In other words, the right eye unit A1R is a unit for integrally moving the projection optical system 30 and the calibration optical system 60 included in the right eye measurement unit 7R, and the right eye deflection mirror 81R. For example, the second unit A2 is a unit for integrally moving the first unit A1 (left eye unit A1L and right eye unit A1R), the reflection mirror 84, and the concave mirror 85.

For example, the first unit A1 is moved in a three-dimensional direction by the first moving unit 9 with respect to a base 86 (base 86L and base 86R) fixed in the second unit A2. Accordingly, for example, the first unit A1 can move in the three-dimensional direction with respect to the concave mirror 85. In addition, by moving at least in one of the X direction and the Y direction in the first unit A1, the incident position of the apparent light flux for forming an image of the target light flux in front of the subject eye with respect to the concave mirror 85 is changed. Further, the movement in the Z direction in the first unit A1 changes the distance between the concave mirror 85 and the first unit A1, and the presentation distance of the image of the target light flux from the measurement unit 7 is changed.

For example, the first moving unit 9 includes an X moving unit, a Y moving unit, a Z moving unit, a driving unit, and the like. As an example, the X moving unit may be a slide mechanism that moves the Y moving unit in the X direction with respect to the base 86. Further, the Y moving unit may be a slide mechanism that moves the Z moving unit in the Y direction with respect to the X moving unit. Further, the Z moving unit may be a slide mechanism that moves the first unit A1 in the Z direction with respect to the Y moving unit. In other words, the first unit A1 is connected to the Z moving unit and is moved in the three-dimensional direction with respect to the base 86 by the movement of the Z moving unit, the movement of the Y moving unit, and the movement of the X moving unit. The driving unit may include a plurality of motors or the like for driving each moving unit.

For example, the first moving unit 9 includes a left eye moving unit 9L for moving the left eye unit A1L and a right eye moving unit 9R for moving the right eye unit A1R. Accordingly, each of the left eye unit A1L and the right eye unit A1R moves independently in the three-dimensional direction. The movement amount of the left eye unit A1L and the right eye unit A1R in each direction is detected by using the number of pulses of the motor or the like.

For example, the second unit A2 is moved in the Z direction by the second moving unit 8 with respect to the base 87 fixed in the housing 2. Accordingly, for example, the second unit A2 can move in the Z direction with respect to the subject eye E. For example, by moving the second unit A2 in the Z direction, the first unit A1 (measurement unit 7) can be moved in the Z direction without changing the distance from the concave mirror 85 to the first unit A1. In other words, the first unit A1 (measurement unit 7) can be moved in the Z direction while maintaining the positional relationship between the concave mirror 85 and the first unit A1. For example, the second moving unit 8 includes the Z moving unit and the driving unit. As an example, the Z moving unit may be a slide mechanism that moves the second unit A2 in the Z direction with respect to the base 87. In other words, the second unit A2 is connected to the Z moving unit and is moved in the Z direction with respect to the base 87 by the movement of the Z moving unit. The driving unit may include a motor or the like for driving the Z moving unit. The movement amount of the second unit A2 in the Z direction is detected by using the number of pulses of the motor or the like.

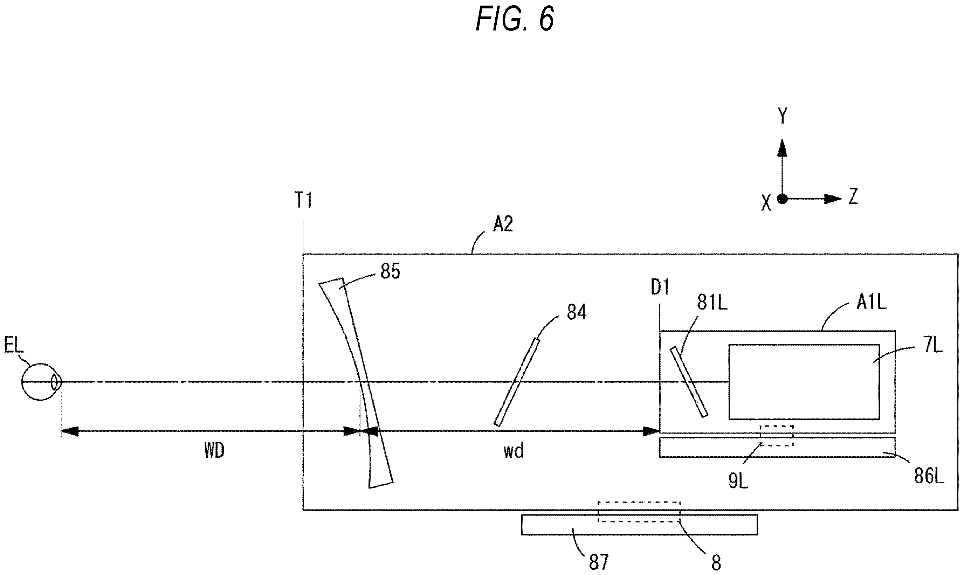

FIG. 6 is a simplified view in which optical members inside the optometry device 100 are arranged on a straight line. For example, the first unit A1 is arranged at an initial position D1 inside the second unit A2. The initial position D1 is a position in the Z direction such that a position of the pupil (that is, pupil position) of the subject eye E and the pupil conjugate position of the subjective measurement unit have a constant positional relationship in a case where the distance from the subject eye E to the concave mirror 85 is adjusted to an appropriate working distance WD. For example, the second unit A2 is arranged at a measurement position T1. The measurement position T1 is a position in the Z direction at which the distance from the subject eye E to the concave mirror 85 is the appropriate working distance WD.

In the present example, by moving the second unit A2 with respect to the subject eye E to arrange the second unit A2 at the measurement position T1, and by keeping the distance between the subject eye E and the concave mirror 85 to be the appropriate working distance WD, the first unit A1 moved together with the second unit A2 is arranged at the initial position D1. At this time, the distance from the subject eye E to the concave mirror 85 (that is, appropriate working distance WD) and a distance wd from the concave mirror 85 to the first unit A1 are the same, and the subject eye E and the deflection mirror 81 are optically conjugated through the concave mirror 85. Further, the optical magnification from the subject eye E to the concave mirror 85 and the optical magnification from the concave mirror 85 to the first unit A1 become the same magnification. Accordingly, the image of the target light flux is satisfactorily irradiated onto the subject eye E.

### <Control unit>

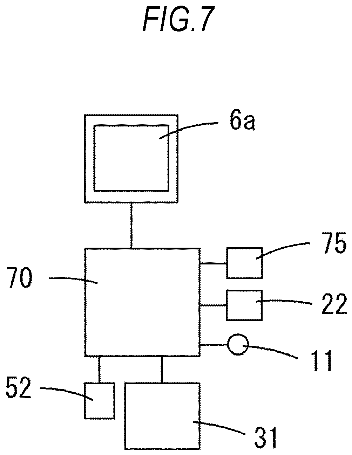

FIG. 7 is a view showing a control system of the optometry device 100. The control unit 70 includes a CPU (processor), a RAM, a ROM, and the like. For example, the CPU controls each member of the optometry device 100. For example, the RAM temporarily stores various pieces of information. For example, various programs for controlling the operation of the optometry device 100, the target, initial values, and the like are stored in the ROM. The control unit 70 may be include a plurality of control units (that is, a plurality of processors).

For example, various members such as the monitor 6a, the light source 11, the imaging element 22, the display 31, the imaging element 52, and the non-volatile memory 75 (hereinafter referred to as a memory 75) are electrically connected to the control unit 70. Further, for example, the control unit 70 is electrically connected to the driving unit of the first moving unit 9, the driving unit of the second moving unit 8, the drive mechanism 39, and the like. For example, the memory 75 is a non-transitory storage medium that can hold stored contents even when power supply is interrupted. For example, as the memory 75, a hard disk drive, a flash ROM, a USB memory, or the like can be used.

### <Control operation>

The control operation of the optometry device 100 will be described.

### <Alignment of subject eye>

The examiner instructs the examinee to observe the presentation window 3 with face in contact with the forehead pad 4 and the chin support 5. Since the face of the examinee is fixed, the subject eye E is less likely to shift in the X direction, the Y direction, and the Z direction. Subsequently, the examiner operates the switch unit 6b to display the fixation target for fixing the subject eye E on the display 31. Accordingly, the fixation target is irradiated to the subject eye E.

Furthermore, the examiner operates the switch unit 6b to select a switch for performing alignment with respect to the subject eye E. The control unit 70 controls the first index light projecting optical system 45, the second index light projecting optical system 46, and the observation optical system 50 according to the input signal from the switch unit 6b, projects an alignment index onto the cornea of the subject eye E, and acquires the anterior eye part image including the alignment index image of the subject eye E. Further, the control unit 70 detects the corneal apex position using the alignment index image of the anterior eye part image, and the first unit A1, and moves the second unit A2 with respect to the alignment reference position in the X direction, the Y direction, and the Z direction based on the displacement of the corneal apex position.

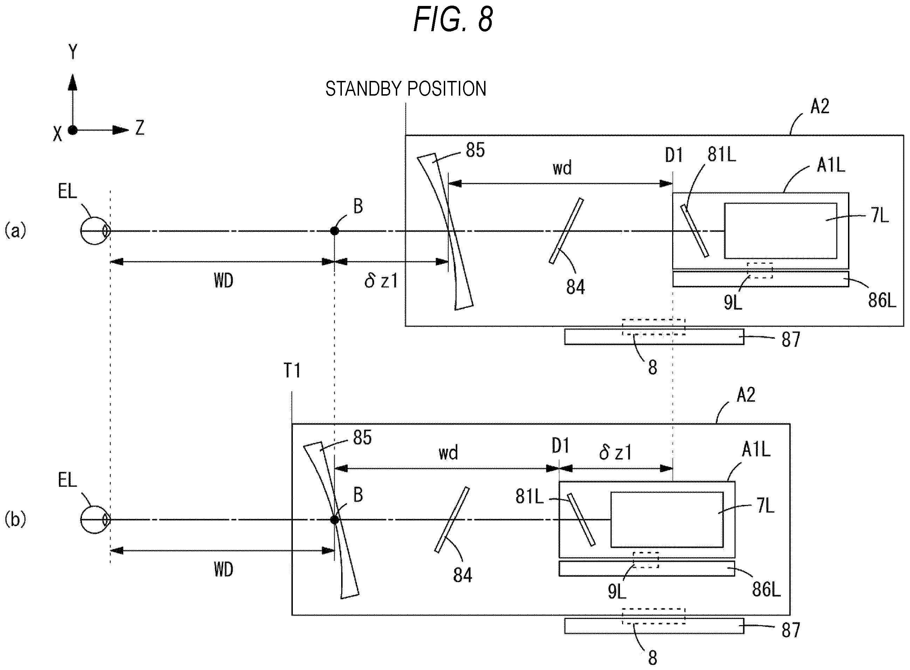

First, the movement of each unit in the Z direction will be described. FIG. 8 is a view showing positions of the first unit A1 and the second unit A2 with respect to the subject eye E. The state (a) illustrated in FIG. 8 shows a state where the second unit A2 is in the standby position with respect to the subject eye E. The state (b) illustrated in FIG. 8 shows a state where the second unit A2 is in the measurement position T1 with respect to the subject eye E. As shown in the state (a) illustrated in FIG. 8, the second unit A2 is positioned in the standby position at the start of alignment of the subject eye E. For example, at this time, the concave mirror 85 is positioned to be away by a distance δz1 in the Z direction from a position B where the distance between the subject eye E and the concave mirror 85 is set to the appropriate working distance WD. The distance δz1 may change depending on the shape of the subject eye E (for example, the deep set eyes or the protruding eyes). Further, since there is a difference of the distance δz1 between the distance from the subject eye E to the concave mirror 85 (that is, working distance WD + distance δz1) and the distance wd from the concave mirror 85 to the first unit A1, the target is presented to the subject eye E at a magnification different from the predetermined optical magnification.

The control unit 70 controls the driving unit of the second moving unit 8 to make the subject eye E and the concave mirror 85 match each other at the appropriate working distance WD, and moves the second unit A2 in the Z direction with respect to the base 87. Further, when the displacement of the corneal apex position in the Z direction falls within the allowable range, the control unit 70 stops the control of the driving unit of the second moving unit 8 to stop the movement of the second unit A2. Accordingly, as shown in the state (b) illustrated in FIG. 8, the second unit A2 moves by the distance δz1 with respect to the subject eye E and is arranged at the measurement position T1, and the concave mirror 85 is arranged at the position B. The subject eye E and the concave mirror 85 match each other at the appropriate working distance WD, and the alignment of the first unit A1 (measurement unit 7) with respect to the subject eye E in the Z direction is completed.

The first unit A1 is positioned at the initial position D1 through the base 86, inside the second unitA2. The first unit A1 moves by the distance δz1 with the movement of the second unit A2, and the distance from the subject eye E to the concave mirror 85 (working distance WD) and the distance wd from the concave mirror 85 to the first unit A1 are adjusted to the same distance. Therefore, the target is presented to the subject eye E at a predetermined optical magnification.

Next, the movement of the first unit A1 in the X direction and the Y direction will be described. The position of the subject eye E changes depending on the pupillary distance, the convergence angle (specifically, the convergence angle corresponding to the optical presentation distance of the target), and the like. In order to appropriately guide the target light flux to the subject eye E, it is necessary to change the incident position of the target light flux in the XY direction with respect to the concave mirror 85 based on the position of the subject eye E. In other words, it is necessary to make the optical axis match with the subject eye E. When there is a displacement in the XY direction between the subject eye E and the optical axis, there is a possibility that the subject eye E cannot visually recognize the target satisfactorily. For example, problems that a part of the target appearing to be missing or the target cannot be fused well with the left and right eyes may occur.

The control unit 70 controls the driving unit of the first moving unit 9 to make the optical axis match with the subject eye E, and moves the first unit A1 in the X direction and the Y direction with respect to the base 86. Further, when the displacement of the corneal apex position in the X direction and the Y direction falls within the allowable range, the control unit 70 stops the control of each driving unit of the first moving unit 9 to stop the movement of the first unit A1. Accordingly, the first unit A1 is moved in the XY direction with respect to the concave mirror 85, the subject eye E and the optical axis match each other, and the alignment of the first unit A1 (measurement unit 7) with respect to the subject eye E in the XY direction is completed. The subject eye E can appropriately visually recognize the target.

### <Subjective measurement>

When the alignment of the subject eye E and the first unit A1 is completed, the examiner starts the subjective measurement for the subject eye E. The examiner operates the switch unit 6b to set the calibration power for calibrating the subject eye E. As an example, the spherical power, the cylindrical power, and the astigmatism axis angle for calibrating the subject eye E may be selected based on the objective eye refractive power (objective value) of the subject eye E.

The control unit 70 controls at least one of the projection optical system 30 and the calibration optical system 60 according to the selection signal from the switch unit 6b. For example, the control unit 70 may calibrate the spherical power of the subject eye E by driving the drive mechanism 39 and moving the display 31 in the optical axis L2 direction. Further, for example, by driving the rotation mechanism 62a and the rotation mechanism 62b and rotating the cylindrical lens 61a and the cylindrical lens 61b around the optical axis L2b, the control unit 70 may calibrate at least one of the cylindrical power and the astigmatism axis angle of the subject eye E. Accordingly, the subject eye E is calibrated by a predetermined diopter value (for example, 0 D or the like).

The examiner operates the switch unit 6b to confirm whether the calibration power for calibrating the subject eye E is appropriate while switching the visual acuity value of the target presented to the subject eye E. For example, when the examinee is asked to answer the direction of the target and the examinee answers correctly, the visual acuity value is switched to a one-step higher visual acuity value (that is, a small value), and when the examinee answers incorrectly, the visual acuity value is switched to a one-step lower visual acuity value (that is, a large value). The control unit 70 changes the target displayed on the display 31 based on the change signal from the switch unit 6b. In addition, when the calibration power for calibrating the subject eye E is inappropriate, the calibration power is changed. Accordingly, the subjective eye refractive power (subjective value) of the subject eye E is acquired.

### <Tracking of subject eye>

In the above, the alignment is completed before the subjective measurement of the subject eye E is started, but the relationship between the subject eye E and the first unit A1 is not always maintained during the subj ective measurement. For example, when the subj ect eye E moves or the presentation distance of the target is changed, there is a possibility that the corneal apex position is displaced from the alignment reference position and is out of the allowable range. Therefore, the control unit 70 may always detect the displacement even during the subjective measurement, and when the corneal apex position is displaced from the allowable range, the first unit A1 may track the subject eye E.

In the present example, the first moving unit 9 is controlled according to the fine movement of the subject eye E, and only the first unit A1 is moved to track the subject eye E. Regarding the movement of the subject eye in the Z direction, it is also possible to move the second unit A2 by controlling the second moving unit 8, but it is difficult for the second unit A2 to move quickly because there are many optical members that integrally move and are obstructive in a large scale.

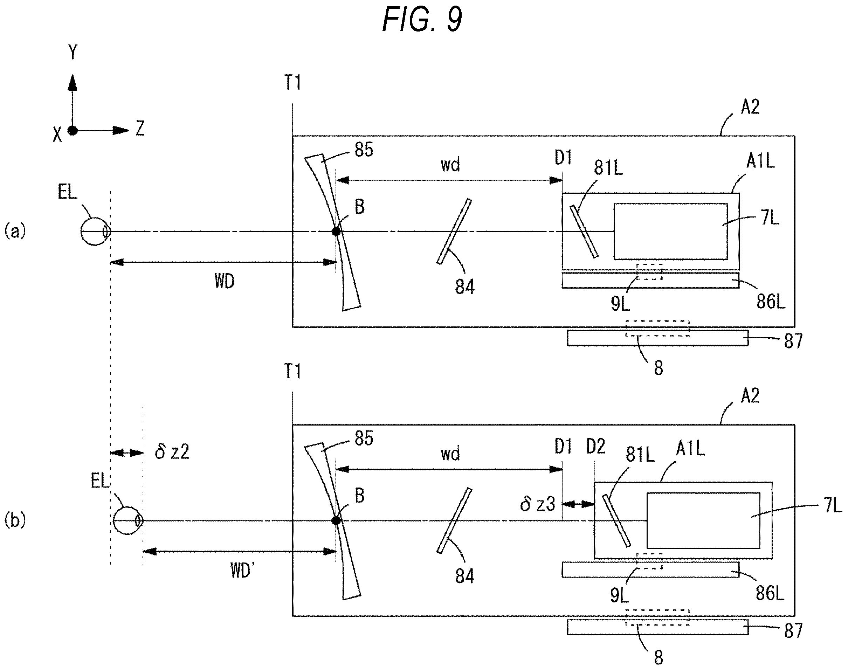

FIG. 9 is a view showing the position of the first unit A1 with respect to the concave mirror 85. The state (a) illustrated in FIG. 9 shows a state where the first unit A2 is in the initial position D1. The state (b) illustrated in FIG. 9 shows a state where the first unit A1 is in the moving position D2 moved from the initial position D 1. In the middle of the subjective measurement, the first unit A1 is moved in at least one of the X direction, the Y direction, and the Z direction according to the displacement of the corneal apex position, but here, for convenience, the Z direction will be described.

For example, when the subject eye E moves from the state (a) illustrated in FIG. 9 toward the concave mirror 85 by a distance δz2, the state (b) illustrated in FIG. 9 is obtained, and the distance from the subject eye E to the concave mirror 85 is changed to a working distance WD'. At this time, the control unit 70 controls the driving unit of the first moving unit 9 and moves the first unit A1 with respect to the base 86 in a direction away from the concave mirror 85 by a distance δz3. The relationship between the displacement amount of the subject eye E in the Z direction and the movement amount of the first unit A1 in the Z direction may be preset. Accordingly, the optical presentation distance of the target to the subject eye E is maintained. Similarly, when the subject eye E moves in the X direction and the Y direction, the control unit 70 controls the driving unit of the first moving unit 9, and the first unit A1 is moved with respect to the base 86 in the X direction and Y direction of the concave mirror 85. The relationship between the displacement amount of the subject eye E in the XY direction and the movement amount of the first unit A1 in the XY direction may be preset. Accordingly, the subject eye E and the optical axis match each other, and the subject eye E can appropriately visually recognize the target.

### <Correction of optical aberration>

In the tracking of the subject eye E, by moving the first unit A1 with respect to the concave mirror 85, the relationship of the optical magnifications in the distance from the subject eye E to the concave mirror 85 (working distance WD') and the distance from the concave mirror 85 to the first unit A1 (distance wd + distance δz3) is slightly broken. Therefore, optical aberrations may occur, and an appropriate target may not be presented to the subject eye E.

Therefore, the control unit 70 uses the position information of the first unit A1 in each direction to set the correction amount for correcting the change at the optical magnification. In other words, the correction amount for correcting the change in the optical magnification is set based on the movement amount in each direction from the initial position D1 to the movement position D2. As an example, a correction amount for correcting the optical magnification to 1 may be set. For example, the memory 75 may store a correction table based on an experiment or simulation in which the correction amount is associated with the position information of the first unit A1.

For example, the control unit 70 may control the display of the display 31 and change the size of the target based on the correction amount acquired from the correction table. Accordingly, it is possible to present an appropriate target in consideration of the optical aberrations due to the movement of the first unit A1.

As described above, for example, in the subjective optometry device in the present example, the first moving means for moving the measurement unit including at least the projection optical system with respect to the fixed optical member, and the second moving means for moving the measurement unit and the fixed optical member with respect to the subject eye in a state where the positional relationship between the measurement unit and the fixed optical member is maintained are controlled in the alignment of the subject eye and the measurement unit. For example, in the alignment of the subject eye and the optical axis of the projection optical system, it is possible to reduce the occurrence of optical aberrations due to the movement of the measurement unit by controlling the first moving means. Further, for example, in the aligning for projecting the image of the target light flux onto the subject eye at a predetermined optical magnification, the change in the optical magnification can be reduced by controlling the second moving means. As a result, the optical characteristic of the subject eye can be measured accurately.

Further, for example, the subjective optometry device in the present example performs the alignment by controlling the second moving means, and then performs the alignment by controlling the first moving means. For example, by controlling the second moving means first and adjusting the optical magnification to a predetermined optical magnification in advance, the movement of the measurement unit is minimized in controlling the first moving means, and the optical aberrations that may occur becomes smaller. For example, when the first moving means is controlled first, the measurement unit moves significantly with respect to the fixed optical member, and thus the optical aberrations are likely to increase. Further, in order to reduce the optical aberrations by controlling of the second moving means, it is necessary to move the first moving means again. As an example, after moving the measurement unit in the forward direction, it becomes necessary to move the measurement unit in the rearward direction in consideration of the integrated movement amount of the measurement unit and the fixed optical member in the forward direction. Therefore, the control is likely to be complicated.

Further, for example, the subjective optometry device according to the present example controls the second moving means to perform the rough alignment of the measurement unit with respect to the subject eye and controls the first moving means to perform the fine alignment of the measurement unit with respect to the subject eye, to adjust alignment between the subject eye and the measurement unit. By controlling the second moving means to be matched to a predetermined optical magnification and then controlling the first moving means to make the subject eye and the optical axis of the projection optical system match each other, an appropriate target can be presented to the subject eye.

Further, for example, the subjective optometry device according to the present example performs alignment (rough alignment and fine alignment) between the subject eye and the measurement unit, and then performs tracking of the subject eye by controlling the first moving means. For example, by quickly responding to fine movements of the subject eye during the subjective measurement by controlling the first moving means, an appropriate target is presented to the subject eye.

Further, for example, the subj ective optometry device according to the present example moves the measurement unit and the fixed optical member at least in the front-rear direction with respect to the subject eye, and moves the measurement unit at least in the left-right direction and the up-down direction with respect to the fixed optical member. For example, the displacement of the subject eye in the front-rear direction has a large effect on the optical magnification, but by controlling the second moving means, a predetermined optical magnification is likely to be maintained. Further, for example, the occurrence of the displacement of the subject eye in the left-right direction and the up-down direction (that is, the displacement between the subject eye and the optical axis) can be reduced by controlling the first moving means.

Further, for example, the subjective optometry device according to the present example acquires the position information of the measurement unit, and based on this position information, corrects an optical aberration which is generated on an account that the target light flux passes through the fixed optical member. Accordingly, for example, even when the measurement unit is moved with respect to the fixed optical member, the relationship of the optical magnifications is broken, and optical aberrations occurs, the optical axis of the projection optical system is appropriately aligned with the subject eye.

Further, for example, the subjective optometry device in the present example uses a concave mirror as a fixed optical member. For example, the concave mirror optically presents the target at a predetermined examination distance, and it is not necessary to arrange the members that form each optical system at an actual distance. Therefore, the device is miniaturized. Further, for example, when the concave mirror is used as the fixed optical member, large optical aberrations due to the movement of the measurement unit with respect to the fixed optical member are more likely to occur than when a lens or the like is used. However, by providing the first moving means for performing alignment of the optical axis of the projection optical system with the subject eye and the second moving means for performing alignment of the image of the target light flux to be irradiated onto the subject eye at a predetermined optical magnification, the measurement unit can be moved to reduce the influence of the optical aberrations to be smaller.

### <Modification example>

Further, in the present example, as the first unit A1, the configuration in which the deflection mirror 81 and the measurement unit 7 are integrally moved with respect to the concave mirror 85 has been described as an example, but the present invention is not limited thereto. For example, only the measurement unit 7 may be moved with respect to the concave mirror 85. In this case, a driving unit that can move the measurement unit 7 in the X direction, the Y direction, and the Z direction may be provided, and the measurement unit 7 may be moved in accordance with the rotation of the deflection mirror 81. As an example, the measurement unit 7 is moved in the X direction and the Y direction with respect to the deflection mirror 81, and accordingly, the incident position of the target light flux (the optical axis of the target light flux) may be matched with the position according to the pupillary distance and the convergence angle of the subject eye E. Further, the measurement unit 7 is moved in the Z direction with respect to the deflection mirror 81, and accordingly, the display distance of the target may be changed.

Further, in the present example, the configuration in which the second moving unit 8 can move the second unit A2 in the Z direction has been described as an example, but the present invention is not limited thereto. A configuration in which the second moving unit 8 can move the second unit A2 at least in the Z direction may be employed. This is because the position of the subject eye E in the Z direction changes depending on the shape of the subject eye E (deep set eyes or protruding eyes). For example, with such a configuration, the optical magnification can be maintained regardless of the shape of the subject eye E. The positions of the subject eye E in the X direction and Y direction are approximately fixed by the forehead pad 4 and the chin support 5, but it is needless to say that the second unit A2 may move in the X direction and Y direction.

Further, in the present example, the configuration in which the second moving unit 8 integrally moves the concave mirror 85 and the first unit A1 in the second unit A2 has been described as an example, but the present invention is not limited thereto. A configuration in which the second moving unit 8 substantially integrally moves the concave mirror 85 and the first unit A1 may be employed. For example, in this case, the second moving unit 8 may include a driving unit and a Z moving unit for moving the concave mirror 85 in the Z direction, and a driving unit and a Z moving unit for moving the first unit A1 in the Z direction. By controlling each driving unit such that the movement amount of the concave mirror 85 and the movement amount of the first unit A1 are the same, the second moving unit 8 may maintain the positional relationship between the concave mirror 85 and the first unit A1, and a predetermined optical magnification may be maintained.

Further, in the present example, the configuration in which the first unit A1 is moved in the X direction and Y direction by detecting the displacement between the corneal apex position and the alignment reference position using the anterior eye part image obtained by capturing the anterior eye part in the alignment of the subject eye E has been described as an example, but the present invention is not limited thereto. For example, the first unit A1 may be moved in the X direction and the Y direction by detecting the pupillary distance using a face image obtained by capturing the face of the examinee. Further, for example, alignment may be performed by using both of the anterior eye part image and the face image. In this case, the first unit A1 may be roughly aligned in the XY direction according to the pupillary distance based on the face image, and the first unit A1 may be finely aligned in the XY direction according to the displacement from the alignment reference position based on the anterior eye part image.

Further, in the present example, in the alignment of the subject eye E, a configuration in which the second unit A2 is moved in the Z direction and then the first unit A1 is moved in the XY direction has been described as an example, but the present invention is not limited thereto. For example, the second unit A2 may be moved in the Z direction while the first unit A1 may be moved in the XY direction. Accordingly, alignment can be performed efficiently.

Further, in the present example, the configuration in which the face of the examinee is fixed by the forehead pad 4 and the chin support 5 has been described as an example, but the present invention is not limited thereto. For example, the face of the examinee may be fixed only by the forehead pad 4. For example, in the subjective measurement that is performed based on the response of the examinee, the subject eye E is likely to move particularly in the Y direction when the examinee speaks with the chin in contact with the chin support 5. Such movement in the Y direction may be reduced by using only the forehead pad 4 without using the chin support 5.

## Claims

1. A subjective optometry device for subjectively measuring an optical characteristic of a subject eye (E), the subjective optometry device comprising:
- a projection optical system (30) that projects a target light flux toward the subject eye (E);
- a calibration optical system (60) that changes an optical characteristic of the target light flux;
- a fixed optical member that is fixedly arranged in an optical path of the projection optical system (30) and optically presents an image of the target light flux to the subject eye (E) at a predetermined examination distance; and
- a first moving means (9) for moving a measurement unit (A1) with respect to the fixed optical member to perform first alignment of an optical axis of the projection optical system (30) with respect to the subject eye (E), the measurement unit (A1) including at least the projection optical system (30);
**characterized by**:
- a second moving means (8) for moving the measurement unit (A1) and the fixed optical member with respect to the subject eye (E), in a state of maintaining a positional relationship between the measurement unit (A1) and the fixed optical member, to perform second alignment such that the image of the target light flux is irradiated onto the subject eye (E) at a predetermined optical magnification; and
- a movement control means (70) for controlling the first moving means (9) and the second moving means (8) to perform alignment of the measurement unit (A1) with respect to the subject eye (E).

2. The subjective optometry device according to claim 1, wherein the movement control means (70) controls the first moving means (9) to perform the first alignment after controlling the second moving means (8) to perform the second alignment.

3. The subjective optometry device according to claim 1 or 2, wherein the movement control means (70)
- controls the second moving means (8) to perform rough alignment of the measurement unit (A1) with respect to the subject eye (E) and
- controls the first moving means (9) to perform fine alignment of the measurement unit (A1) with respect to the subject eye (E), to adjust alignment between the subject eye (E) and the measurement unit (A1).

4. The subjective optometry device according to claim 3, wherein the movement control means (70) controls the first moving means (9) to perform tracking of the subject eye (E) after performing the alignment between the subject eye (E) and the measurement unit (A1).

5. The subjective optometry device according to any one of claims 1 to 4, wherein the movement control means (70)
- controls the second moving means (8) to move the measurement unit (A1) and the fixed optical member at least in a front-rear direction with respect to the subject eye (E), and
- controls the first moving means (9) to move the measurement unit (A1) at least in a left-right direction and an up-down direction with respect to the fixed optical member.

6. The subjective optometry device according to any one of claims 1 to 5, wherein
- the measurement unit (A1) has a left eye measurement unit (7L) and a right eye measurement unit (7R) provided as a left and right pair,
- the fixed optical member is shared in an optical path of a target light flux from the left eye measurement unit (7L) and in an optical path of a target light flux from the right eye measurement unit (7R), and
- the second moving means (8) integrally moves the left eye measurement unit (7L), the right eye measurement unit (7R), and the fixed optical member.

7. The subjective optometry device according to any one of claims 1 to 6, further comprising:
- a position information acquisition means for acquiring position information of the measurement unit (A1); and
- a correction means for correcting an optical aberration, which is generated on an account that the target light flux passes through the fixed optical member, based on the position information.

8. The subjective optometry device according to any one of claims 1 to 7, wherein
- the measurement unit (A1) includes the calibration optical system (60) with the projection optical system (30), and
- the fixed optical member guides the target light flux calibrated by the calibration optical system (60) to the subject eye (E).

9. The subjective optometry device according to any one of claims 1 to 8, wherein the fixed optical member is a concave mirror.

## Patentansprüche

1. Subjektive optometrische Vorrichtung zum subjektiven Messen einer optischen Eigenschaft eines Objektauges (E), wobei die subjektive optometrische Vorrichtung umfasst:
- ein optisches Projektionssystem (30), das einen Ziellichtstrom in Richtung des Objektauges (E) projiziert;
- ein optisches Kalibrierungssystem (60), das eine optische Eigenschaft des Ziellichtstroms ändert;
- ein befestigtes optisches Element, das fest in einem optischen Pfad des optischen Projektionssystems (30) angeordnet ist und dem Objektauge (E) ein Bild des Ziellichtstroms mit einem vorbestimmten Untersuchungsabstand optisch präsentiert; und
- ein erstes Bewegungsmittel (9) zum Bewegen einer Messeinheit (A1) in Bezug auf das befestigte optische Element, um erste Ausrichtung einer optischen Achse des optischen Projektionssystems (30) in Bezug auf das Objektauge (E) auszuführen, wobei die Messeinheit (A1) zumindest das optische Projektionssystem (30) umfasst;
**gekennzeichnet durch:**
- ein zweites Bewegungsmittel (8) zum Bewegen der Messeinheit (A1) und des befestigten optischen Elements in Bezug auf das Objektauge (E) in einem Zustand, in dem eine positionelle Beziehung zwischen der Messeinheit (A1) und dem befestigten optischen Element gehalten ist, um zweite Ausrichtung auszuführen, sodass das Bild des Ziellichtstroms auf das Objektauge (E) mit einer vorbestimmten optischen Vergrößerung ausgestrahlt wird; und
- eine Bewegungssteuereinheit (70) zum Steuern des ersten Bewegungsmittels (9) und des zweiten Bewegungsmittels (8) zum Ausführen von Ausrichtung der Messeinheit (A1) in Bezug auf das Objektauge (E).

2. Subjektive optometrische Vorrichtung nach Anspruch 1, wobei die Bewegungssteuereinheit (70) das erste Bewegungsmittel (9) zum Ausführen der ersten Ausrichtung nach Steuern des zweiten Bewegungsmittels (8) zum Ausführen der zweiten Ausrichtung steuert.

3. Subjektive optometrische Vorrichtung nach Anspruch 1 oder 2, wobei das Bewegungssteuermittel (70)
- das zweite Bewegungsmittel (8) zum Ausführen von Grobausrichtung der Messeinheit (A1) in Bezug auf das Objektauge (E) steuert und
- das erste Bewegungsmittel (9) zum Ausführen von Feinausrichtung der Messeinheit (A1) in Bezug auf das Objektauge (E) steuert, um Ausrichtung zwischen dem Objektauge (E) und der Messeinheit (A1) einzustellen.

4. Subjektive optometrische Vorrichtung nach Anspruch 3, wobei das Bewegungssteuermittel (70) das erste Bewegungsmittel (9) zum Ausführen von Verfolgung des Subjektauges (E) nach Ausführen der Ausrichtung zwischen dem Subjektauge (E) und der Messeinheit (A1) steuert.

5. Subjektive optometrische Vorrichtung nach einem der Ansprüche 1 bis 4, wobei die Bewegungssteuereinheit (70)
- das zweite Bewegungsmittel (8) zum Bewegen der Messeinheit (A1) und des befestigten optischen Elements zumindest in einer Vorne-Hinten-Richtung in Bezug auf das subjektive Auge (E) steuert, und
- das erste Bewegungsmittel (9) zum Bewegen der Messeinheit (A1) zumindest in einer Links-Rechts-Richtung und einer Aufwärts-Abwärts-Richtung in Bezug auf das befestigte optische Element steuert.

6. Subjektive optometrische Vorrichtung nach einem der Ansprüche 1 bis 5, wobei
- die Messeinheit (A1) eine linke Augenmesseinheit (7L) und eine rechte Augenmesseinheit (7R) umfasst, die als ein linkes und rechtes Paar vorgesehen sind,
- das befestigte optische Element in einem optischen Pfad eines Ziellichtstroms von der linken Augenmesseinheit (7L) und in einem optischen Pfad eines Ziellichtstroms von der rechten Augenmesseinheit (7R) mitbenutzt wird, und
- das zweite Bewegungsmittel (8) integral die linke Augenmesseinheit (7L), die rechte Augenmesseinheit (7R) und das befestigte optische Element bewegt.

7. Subjektive optometrische Vorrichtung nach einem der Ansprüche 1 bis 6, ferner umfassend
- ein Positionsinformation-Akquisitionsmittel zum Akquirieren von Positionsinformation der Messeinheit (A1); und
- ein Korrekturmittel zum Korrigieren einer optischen Aberration, die basierend darauf erzeugt ist, dass der Ziellichtstrom durch das befestigte optische Element passiert, basierend auf der Positionsinformation.

8. Subjektive optometrische Vorrichtung nach einem der Ansprüche 1 bis 7, wobei
- die Messeinheit (A1) das optische Kalibrierungssystem (60) mit dem optischen Projektionssystem (30) umfasst, und
- das befestigte optische Element den durch das optische Abbildungssystem (60) kalibrierten Objektlichtstrom zu dem Objektauge (E) führt.

9. Subjektive optometrische Vorrichtung nach einem der Ansprüche 1 bis 8, wobei das befestigte optische Element ein Hohlspiegel ist.

## Revendications

1. Dispositif d'optométrie subjective pour mesurer subjectivement une caractéristique optique d'un oeil du sujet (E), le dispositif d'optométrie subjective comprenant :
- un système optique de projection (30) qui projette un flux lumineux cible vers l'œil du sujet (E) ;
- un système optique d'étalonnage (60) qui modifie une caractéristique optique du flux lumineux cible ;
- un élément optique fixe qui est agencé de manière fixe dans un chemin optique du système optique de projection (30) et présente optiquement une image du flux lumineux cible à l'œil du sujet (E) à une distance d'examen prédéterminée ; et
- un premier moyen de déplacement (9) pour déplacer une unité de mesure (A1) par rapport à l'élément optique fixe pour effectuer un premier alignement d'un axe optique du système optique de projection (30) par rapport à l'œil du sujet (E), l'unité de mesure (A1) comprenant au moins le système optique de projection (30) ;
**caractérisé par :**
- un second moyen de déplacement (8) pour déplacer l'unité de mesure (A1) et l'élément optique fixe par rapport à l'œil du sujet (E), dans un état de maintien d'une relation de position entre l'unité de mesure (A1) et l'élément optique fixe, pour effectuer un second alignement de sorte que l'image du flux lumineux cible soit irradiée sur l'œil du sujet (E) à un grossissement optique prédéterminé ; et
- un moyen de commande de mouvement (70) pour commander le premier moyen de déplacement (9) et le second moyen de déplacement (8) pour effectuer un alignement de l'unité de mesure (A1) par rapport à l'œil du sujet (E).

2. Dispositif d'optométrie subjective selon la revendication 1, dans lequel le moyen de commande de mouvement (70) commande le premier moyen de déplacement (9) pour effectuer le premier alignement après avoir commandé le second moyen de déplacement (8) pour effectuer le second alignement.

3. Dispositif d'optométrie subjective selon la revendication 1 ou 2, dans lequel le moyen de commande de mouvement (70) :
- commande le second moyen de déplacement (8) pour effectuer un alignement approximatif de l'unité de mesure (A1) par rapport à l'œil du sujet (E), et
- commande le premier moyen de déplacement (9) pour effectuer un alignement précis de l'unité de mesure (A1) par rapport à l'œil du sujet (E), pour ajuster l'alignement entre l'œil du sujet (E) et l'unité de mesure (A1).

4. Dispositif d'optométrie subjective selon la revendication 3, dans lequel le moyen de commande de mouvement (70) commande le premier moyen de déplacement (9) pour effectuer un suivi de l'œil du sujet (E) après avoir effectué l'alignement entre l'œil du sujet (E) et l'unité de mesure (A1).

5. Dispositif d'optométrie subjective selon l'une quelconque des revendications 1 à 4, dans lequel le moyen de commande de mouvement (70) :
- commande le second moyen de déplacement (8) pour déplacer l'unité de mesure (A1) et l'élément optique fixe au moins dans une direction avant-arrière par rapport à l'œil du sujet (E), et
- commande le premier moyen de déplacement (9) pour déplacer l'unité de mesure (A1) au moins dans une direction gauche-droite et une direction haut-bas par rapport à l'élément optique fixe.

6. Dispositif d'optométrie subjective selon l'une quelconque des revendications 1 à 5, dans lequel :
- l'unité de mesure (A1) présente une unité de mesure de l'œil gauche (7L) et une unité de mesure de l'œil droit (7R) prévues en paire gauche et droite,
- l'élément optique fixe est partagé dans un chemin optique d'un flux lumineux cible provenant de l'unité de mesure de l'œil gauche (7L) et dans un chemin optique d'un flux lumineux cible provenant de l'unité de mesure de l'œil droit (7R), et
- le second moyen de déplacement (8) déplace d'un seul tenant l'unité de mesure de l'œil gauche (7), l'unité de mesure de l'œil droit (7R) et l'élément optique fixe.

7. Dispositif d'optométrie subjective selon l'une quelconque des revendications 1 à 6, comprenant en outre :
- un moyen d'acquisition d'informations de position pour acquérir des informations de position de l'unité de mesure (A1) ; et
- un moyen de correction pour corriger une aberration optique, qui est générée du fait que le flux lumineux cible passe à travers l'élément optique fixe, sur la base des informations de position.

8. Dispositif d'optométrie subjective selon l'une quelconque des revendications 1 à 7, dans lequel :
- l'unité de mesure (A1) comprend le système optique d'étalonnage (60) avec le système optique de projection (30), et
- l'élément optique fixe guide le flux lumineux cible étalonné par le système optique d'étalonnage (60) vers l'œil du sujet (E).

9. Dispositif d'optométrie subjective selon l'une quelconque des revendications 1 à 8, dans lequel l'élément optique fixe est un miroir concave.
